# EUROPÄISCHE PATENTSCHRIFT

(11) **EP 1 127 271 B1**
(45) Veröffentlichungstag und Bekanntmachung des Hinweises auf die Patenterteilung: **29.01.2003**
(21) Anmeldenummer: 99957848.7
(22) Anmeldetag: 22.09.1999
(51) Int. Cl.: G01N 29/02, G01N 9/00

(54) **SENSORANORDNUNG UND VERFAHREN ZUR ERMITTLUNG DER DICHTE UND DER VISKOSITÄT EINER FLÜSSIGKEIT**
SENSOR AND METHOD FOR DETERMINING THE DENSITY AND VISCOSITY OF A LIQUID
SYSTEME DE DETECTION ET PROCEDE POUR DETERMINER LA DENSITE ET LA VISCOSITE D'UN LIQUIDE

(30) Priorität: 04.11.1998 DE 19850803
(43) Veröffentlichungstag der Anmeldung: 29.08.2001
(73) Patentinhaber: ROBERT BOSCH GMBH, 70442 Stuttgart (DE)
(72) Erfinder: HAHN, Dietmar, D-70839 Gerlingen (DE); FLIK, Gottfried, D-71229 Leonberg (DE); HERRMANN, Falk, D-71229 Leonberg (DE)
(86) Internationale Anmeldenummer: DE9903023
(87) Internationale Veröffentlichungsnummer: WO00026658

(56) Entgegenhaltungen:
- WO-A-87/02134
- US-A- 5 741 961
- DU J ET AL: "A STUDY OF LOVE-WAVE ACOUSTIC SENSORS" SENSORS AND ACTUATORS A,CH,ELSEVIER SEQUOIA S.A., LAUSANNE, Bd. A56, Nr. 3, 1. September 1996 (1996-09-01), Seiten 211-219, XP000640390 ISSN: 0924-4247 in der Anmeldung erwähnt
- PATENT ABSTRACTS OF JAPAN vol. 1999, no. 13, 30. November 1999 (1999-11-30) & JP 11 211705 A (FUJI KOGYO KK), 6. August 1999 (1999-08-06)

## Beschreibung

### Stand der Technik

Die Erfindung betrifft eine Sensoranordnung zur Ermittlung der Dichte und Viskosität von Flüssigkeiten und ein Verfahren zur Durchführung dieser Ermittlung nach dem Oberbegriff des Hauptanspruchs.

Allgemein wird bei einer Dichtemessung die Masse eines bekannten Flüssigkeitsvolumens mit einfachen Messanordnungen ermittelt. Außerdem kann auch die Resonanzverstimmung zur Bestimmung der Dichte in einem von der untersuchten Flüssigkeit durchströmten Rohr in einer akustischen Messanordnung ermittelt und ausgewertet werden. Zur Viskositätsmessung der Flüssigkeit sind die sogenannte Rotationsviskosimetrie und die Fallkugelviskosimetrie als hinlänglich bekannte Messmethoden anwendbar. Allen genannten Methoden ist gemeinsam, daß die beiden Meßgrößen Dichte und Viskosität mit unterschiedlichen Geräten ermittelt werden müssen, die jeweils einen hohen Raumbedarf haben, bei hohen Anforderungen an die Meßgenauigkeit kostenintensiv sind und zur Messung relativ große Flüssigkeitsvolumina benötigen.

Aufgrund einer immer häufiger werdenden Notwendigkeit der Miniaturisierung und Systemintegration besteht ein Bedarf an kompakten und kostengünstigen Geräten zur hochgenauen online-Dichte- und Viskositätsmessung in kleinen Flüssigkeitsvolumina, der jedoch mit den heute verfügbaren Meßgeräten nicht gedeckt werden kann. Beispiele für eine Anwendung sind die Dichte- und Viskositätsmessung bei der Zumessung von Dieselkraftstoffen in Kraftfahrzeugen, die online-Überwachung des Zustandes von Motorölen oder die Entwicklung mikrofluidischer Analysesysteme in der Chemie oder Medizin, beispielsweise zur Untersuchung physiologischer Medien wie Blut oder Urin oder zur Herstellung pharmazeutischer Produkte.

Mikrosensoren zur Dichte- und Viskositätsmessung von Flüssigkeiten lassen sich aufgrund der zugrunde liegenden Funktionsprinzipien in zwei Kategorien einteilen. Zum einen gibt es sogenannte Surface Acoustic Wave Sensoren (SAW-Sensoren), die unter der Ausnutzung einer Wechselwirkung zwischen der Ausbreitungsstrecke einer akustischen Oberflächenwelle oder einer Bulkwelle und der zu untersuchenden Flüssigkeit arbeiten und zum anderen gibt es Sensoren, deren Messwandler aus resonant schwingenden Mikrostrukturen bestehen.

Bei der gattungsgemäßen Sensoranordnung wird von einem bekannten Messprinzip ausgegangen, das beispielsweise in dem Aufsatz "A study of Love-wave acoustic sensors", J.Du, G.L.Hardling, P.R.Ogilvy und M.Lake in der Fachzeitschrift Sensors and Actuators A56(1996), Seiten 211 bis 219 beschrieben ist. Mit dem hier beschriebenen Messaufbau ist ein Sensor realisiert, bei dem mit horizontal polarisierten akustischen Scherwellen als Oberflächenwellen gearbeitet wird, sog. Leckwellen (Leakywaves) bzw.

Surface Skimming Bulk Wave (SSBW-Wellen) oder Love-Wellen. Diese akustischen Wellenmoden werden mit sogenannten, für sich auch aus dem zuvor erwähnten Stand der Technik bekannten, Interdigitaltransducern erzeugt und auch detektiert, so dass aus dem Ausbreitungsverhalten auf einer Ausbreitungs- oder Messstrecke das gewünschte Sensorsignal gewonnen werden kann.

### Vorteile der Erfindung

Die eingangs erwähnte gattungsgemäße Sensoranordnung zur Ermittlung der Dichte und der Viskosität einer Flüssigkeit ist gemäß der Erfindung mit den kennzeichnenden Merkmalen des Hauptanspruchs und des nebengeordneten Verfahrensanspruches in vorteilhafter Weise weitergebildet.

Diese erfindungsgemäße Sensoranordnung erlaubt durch die Ausnutzung des Einflusses von zusätzlichen, gezielt auf der Sensoroberfläche eines Sensorgrundelements eingebrachten Störungen in einer Ausbreitungsstrecke für die akustischen Wellen in vorteilhafter Weise eine getrennte Messung von Dichte und Viskosität einer Flüssigkeit in einem Messaufbau mit einer hohen Messgenauigkeit. Bei der eingangs genannten bekannten Anordnung ist dagegen bei einer Messung mit Love-Wellenmoden nur die Erfassung eines Dichte-Viskositäts-produkts möglich.

Für sich gesehen ist ein Viskositäts- und Dichtesensor mit einer sogenannten Quarzmikrowaage (QCM - Quarz Crystal Microbalance) für die Messung mit Bulkwellen, also nicht mit Oberflächenwellen, bekannt, bei der ähnliche Störungen in Form von Flüssigkeitsfallen angeordnet sind. Beispielsweise ist dies in dem Aufsatz " Measuring Liquid Properties with Smooth- and Textured-Surface Resonators", S.J.Martin et al. aus IEEE 1993 International Freguency Control Symposium, Seiten 603 bis 608, beschrieben. Hierbei ist beispielsweise die Oberfläche des einen Schwingers mit senkrecht zur Schwingrichtung ausgerichteten Wällen aus Metall, z.B. Gold, versehen. Die Taschen zwischen den Wällen dienen als Flüssigkeitsfallen, wobei die darin befindliche Flüssigkeit die Schwingungsbewegung unabhängig von ihrer Viskosität ausführt.

Bei dieser bekannten Quarzmikrowaage handelt es sich um einen Dickenscherschwinger, der unter Nutzung des inversen piezoelektrischen Effektes durch flächige Elektroden angeregt wird. Da es in einer Flüssigphase aufgrund der Scherbewegung zu keiner direkten Abstrahlung akustischer Energie kommt, denn Schermoden sind in Flüssigkeiten nicht ausbreitungsfähig, ist die QCM auch zur Flüssigkeitsuntersuchung geeignet. Hierbei wird häufig eine Resonanzfrequenzänderung durch Massenanlagerung gemessen, wobei die QCM als frequenzbestimmendes Element in einer Oszillatorschaltung dient.

Gemäß der Erfindung wird in vorteilhafter Weise der Effekt ausgenutzt, dass es zusätzlich in viskosen Flüssigkeiten durch viskose Kopplung zu einer von Viskosität und Dichte der Flüssigkeit bedingten Frequenzverschiebung kommt. Dies kann zur Ermittlung des Dichte-Viskositätsprodukts der Flüssigkeit herangezogen werden, wobei jedoch zusätzlich der Dichte- vom Viskositätseinfluss mit dem erfindungsgemäß vorgeschlagenen Aufbau auch trennbar ist und so beide Größen unabhängig voneinander zu messen sind.

Es werden somit in Weiterbildung der gattungsgemäßen Anordnung mindestens zwei in ihrem Aufbau parallel betriebene Sensorgrundelemente in vorteilhafter Weise verwendet, wobei die Vorteile bei der Verwendung der Oberflächenwellen, insbesondere der SSB-Wellen oder der Love-Wellen ausgenutzt werden können. Diese sind vor allem eine hohe Messempfindlichkeit, die Verwendung von gegenüber der Flüssigkeit geschützten Wandler-Elektroden, eine inerte Oberfläche und eine geringe Querempfindlichkeit.

Gegenüber der Verwendung der bekannten QCM's, kann bei der erfindungsgemäßen Anordnung auf die galvanische Anbringung von Gold verzichtet und die Sensoranordnung insgesamt in einem halbleiterkompatiblen Fertigungsprozess hergestellt werden. Da das bei der bekannten Anordnung mit QCM's verwendete Gold eine sehr hohe Dichte gegenüber der Flüssigkeit aufweist, kann mit dem erfindungsgemäßen Aufbau, dessen Materialien näher an der Dichte der Flüssigkeit liegen, auch demgegenüber eine Erhöhung der Messempfindlichkeit erreicht werden.

Mit dem beanspruchten Messverfahren kann auf einfache Weise über die Auswertung von Frequenzverschiebungen ein leicht weiterzuverarbeitendes Messsignal gewonnen werden. Die Frequenzverschiebungen des Sensorgrundelements mit den Flüssigkeitsfallen weist zusätzlich zum Einfluss des Dichte-Viskositäts-Produkts eine nur durch die Dichte der Flüssigkeit und das effektive Volumen der Flüssigkeitsfallen gegebene Abhängigkeit auf. Werden nun die Frequenzverschiebungen beider Sensorgrundelemente miteinander verknüpft, können Dichte und Viskosität der Messflüssigkeit getrennt ermittelt werden.

Mit der vorliegenden Erfindung wird ein Mikrosensor vorgeschlagen, mit dem die Bestimmung von Dichte und Viskosität von Flüssigkeitsvolumina im ml-Bereich mit hoher Auflösung und Messgenauigkeit möglich ist. Dieser Sensor kann mit zur Massenfertigung geeigneten Batchprozessen kostengünstig hergestellt werden, wobei auf aus der Halbleiterfertigung bekannte Verfahren zurückgegriffen wird. Es wird somit eine Zusammenführung der Vorteile der Sensoren, die unter Ausnutzung der Wechselwirkung zwischen der Ausbreitungsstrecke einer akustischen Oberflächenwelle und der zu untersuchenden Flüssigkeit ein Messsignal erzeugen und anderer Sensoren (z.B. Bulk-Mode-Sensoren = QCM) ermöglicht, wobei die jeweils spezifischen Nachteile vermieden sind.

Diese und weitere Merkmale von bevorzugten Weiterbildungen der Erfindung gehen außer aus den Ansprüchen, einschließlich der rückbezogenen Unteransprüche, auch aus der Beschreibung und den Zeichnungen hervor, wobei die einzelnen Merkmale jeweils für sich allein oder zu mehreren in Form von Unterkombinationen bei der Ausführungsform der Erfindung und auf anderen Gebieten verwirklicht sein und vorteilhafte sowie für sich schutzfähige Ausführungen darstellen können, für die hier Schutz beansprucht wird.

### Zeichnung

Ausführungsbeispiele der erfindungsgemäßen Sensoranordnung werden anhand der Zeichnung erläutert. Es zeigen:
Figur 1 eine schematische Ansicht einer Sensoranordnung zur Ermittlung der Dichte und der Viskosität einer durch die Sensoranordnung strömenden Flüssigkeit;
Figur 2 eine Detailansicht eines Interdigitaltransducers zur Erzeugung und Detektion akustischer Wellen;
Figuren 3 bis 5 Anordnungsvarianten der Interdigitaltransducer nach der Figur 2;
Figuren 6 bis 8 Schnitte durch ein Substrat der Sensoranordnung mit unterschiedlichen Ausführungen der Beschichtung;
Figur 9 eine detaillierte Draufsicht auf zwei Ausbreitungsstrecken der Messflüssigkeit in der Sensoranordnung;
Figur 10 ein Prinzipschaltbild einer mit der Sensoranordnung gekoppelten Auswerteschaltung und
Figur 11 eine gegenüber der Figur 10 erweiterte Auswerteschaltung.

### Beschreibung der Ausführungsbeispiele

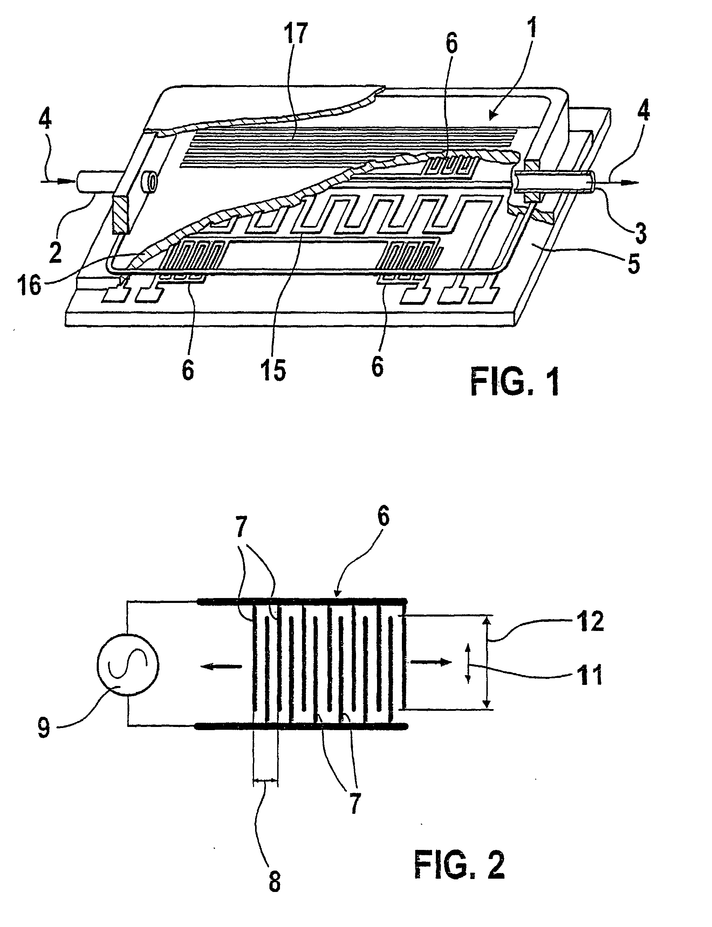

Aus Figur 1 ist eine Sensoranordnung 1 in einer aufgeschnittenen Prinzipdarstellung gezeigt, durch die eine Messflüssigkeit zur Bestimmung ihrer Dichte und ihrer Viskosität von einem Eingang 2 zu einem Ausgang 3 gemäß Pfeil 4 fließt. Hauptbestandteil der vorgeschlagenen Sensoranordnung 1 ist ein einseitig poliertes Substrat 5 aus einem piezoelektrischen Werkstoff, in dem horizontal polarisierte akustische Schermoden von Sensorgrundelementen anregbar und ausbreitungsfähig sind. Als Substratwerkstoffe sind Y-rotierte Quarzschnitte, einige Lithiumniobat- und Lithiumtantalatschnitte sowie entsprechend gepolte piezoelektrische Keramiken geeignet.

Auf der polierten Oberfläche des Substrats 5 befindet sich eine Anordnung aus metallischen Interdigitaltransducern (IDT) 6, die anhand von Figur 2 näher erläutert werden. Diese Interdigitaltransducer 6 sind beispielsweise aus Aluminium, Titan, Chrom, Gold oder Platin, gegebenenfalls auf einer Haftschicht aus Titan oder Silizium und dienen zur Anregung und Detektion der akustischen Oberflächenwellen.

In der Figur 2 ist einer der Interdigitaltransducer 6 im Detail gezeigt, wobei Wandlerfinger 7 akustische Wellen mit der Wellenlänge 8 (Mittenfrequenz) bei einer Anregung durch eine elektrische Spannung an einem Eingang 9 erzeugen können. Hierdurch entsteht eine akustische Oberflächenwelle, d.h. insbesondere eine Scherwelle in der Polarisationsrichtung des Pfeiles 11, mit der Apertur gemäß Pfeil 12. Gemäß eines hier nicht dargestellten Ausführungsbeispiels können die Wandlerfinger 7 auch innerhalb der Periode in zwei Einzelfinger oder auch Splittinger aufgeteilt sein, so dass λ/8 Finger entstehen. Zwischen der elektrischen und der mechanischen Periode liegt hier der Faktor zwei, so dass eine Beseitigung oder zumindest eine Verminderung innerer Reflexionen und des sog. Triple-Transit-Echos (TTE) erreichbar ist.

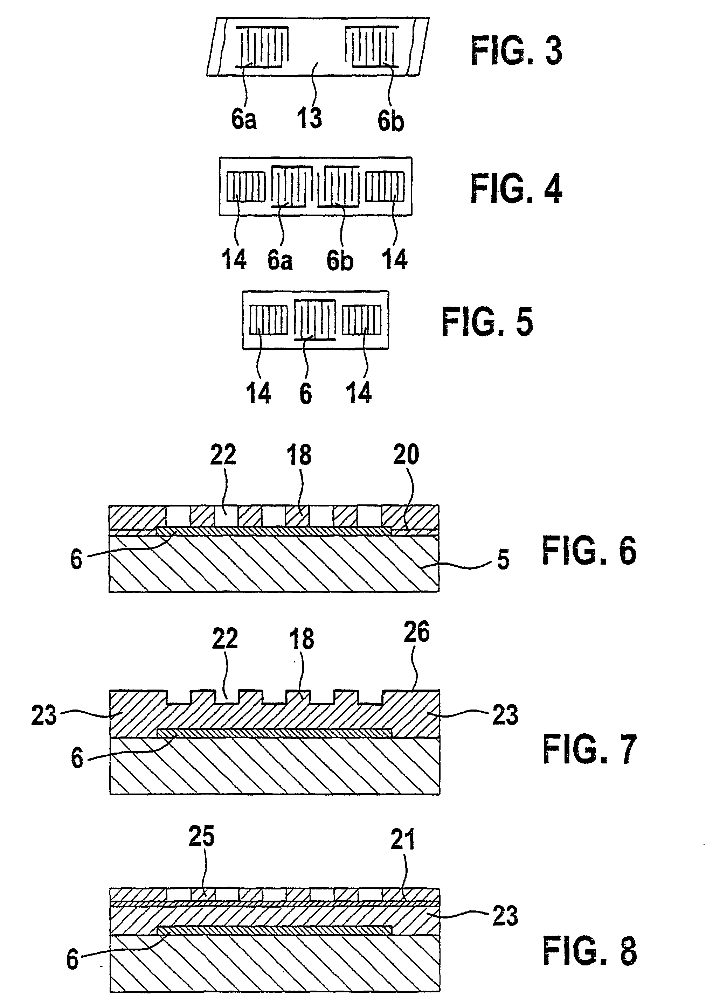

Die Anordnung der Interdigitaltransducer 6 in der Sensoranordnung 1 gemäß der Figur 1 kann nach den Ausführungsbeispielen der Figur 3 bis 5 ausgeführt werden. Beispielsweise nach der Figur 3 als Verzögerungsleitung mit einem Sende-IDT 6a, einer Ausbreitungsstrecke 13 und einem Empfangs-IDT 6b bzw. nach der Figur 4 als Zwei- oder nach der Figur 5 als Eintorresonator mit einem oder zwei IDT 6 und mit Reflektorbänken 14 ausgebildet sein.

Die Sensoranordnung 1 nach der Figur 1 enthält zwei parallel zueinander angeordnete Grundelemente mit den Interdigitaltransducern 6, wobei allerdings zur vereinfachten Auswertung und verbesserten Temperaturkompensation der Messsignale auch ein drittes paralleles, anhand dieser Figur nicht dargestelltes Sensorgrundelement angeordnet werden kann. Weiterhin ist beim Ausführungsbeispiel nach der Figur 1 neben oder zwischen den Grundelementen mit den IDT 6 auf der Oberfläche des Substrats 5 ein mäanderfömiger Dünnschicht-Temperaturwiderstand 15 angeordnet, da insbesondere die Viskosität stark temperaturabhängig ist und somit die Temperatur eine weitere wichtige Messgröße darstellt. Als Material für den Dünnschicht-Temperaturwiderstand 15 kommt hier in vorteilhafter Weise, das Material wie für die IDT 6 in Frage, nämlich Titan/Platin oder Titan/Platin/Titan, wobei die Haftschicht entweder Titan oder auch Silizium sein kann.

Auf dem Substrat 5 nach der Figur 1 ist oberhalb der Grundelemente mit den IDT 6 eine akustische Wellenleiterschicht 16 angeordnet, die z.B. aus einem Ormocer, aus einer Siliziumverbindung oder einem Polymer bestehen kann, so dass aus dem allgemeinen Schermode (Leckwelle oder SSBW) der akustischen Welle ein sog. Wellenleitermode (hier eine Love-Welle) wird. Zur Trennung des Dichteeinflusses vom Viskositätseinfluss bei der Messung werden oberhalb eines Grundelementes mit den IDT 6 gezielt mechanische Störungen in Form von Flüssigkeitsfallen 17 angeordnet, innerhalb derer die akustische Welle aufgrund der mechanischen Inhomogenität nicht ausbreitungsfähig ist.

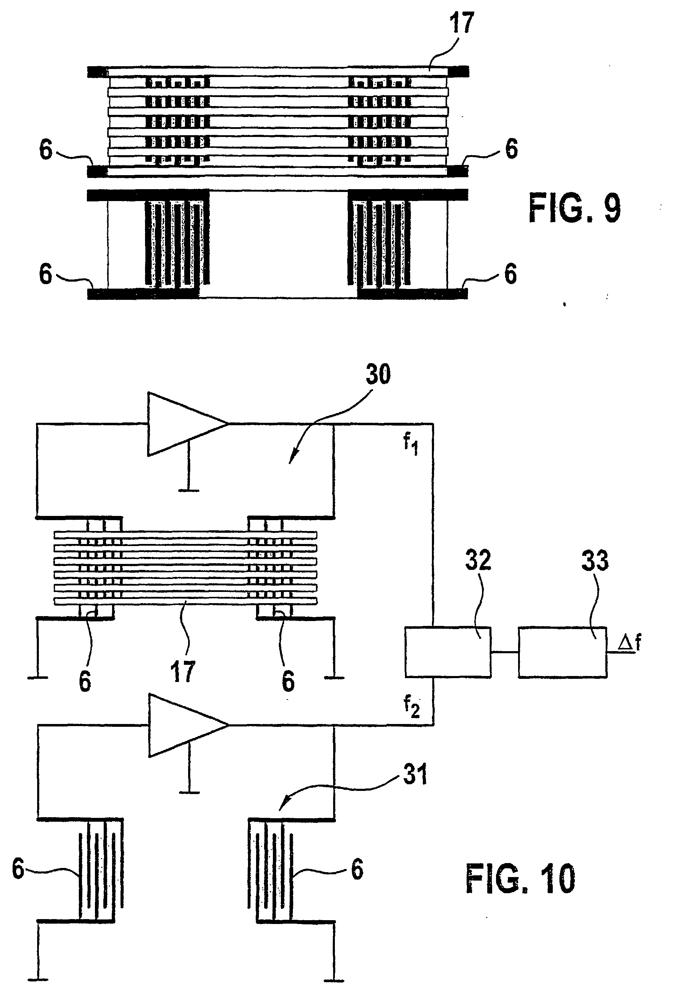

Hierzu wird der Bereich vor, über und zwischen den jeweiligen Interdigitaltransducern 6 mit den parallel zur Ausbreitungsrichtung der akustischen Welle ausgerichteten Wällen 18 versehen, deren geometrische Anordnungsmöglichkeiten anhand von Figuren 6 bis 8 bzw. in der Draufsicht nach Figur 9 erläutert wird. Diese Flüssigkeitsfallen 17 können hier durch entsprechende Strukturierung einer oberhalb des Interdigitaltransducers 6 befindlichen Schicht 20 als Gräben 22 hergestellt werden, wie in der Figur 6 gezeigt oder auch, wie nicht gezeigt, als Gruben oder Schwämme. Zwischen der Schicht 20 und den IDT 6 kann zur Haftungsverbesserung und/oder zum Schutz der IDT 6 eine weitere Zwischenschicht 21 vorgesehen werden. Bei der Anordnung nach der Figur 6 werden die sog. Leckwellen oder SSB-Wellen benutzt.

Wenn es sich um ein sog. Love-Mode-Bauelement handelt, können die Flüssigkeitsfallen nach der Figur 7 auch durch Einbringung grabenartiger Ätzungen 22 direkt in eine Wellenleiterschicht 23 erzeugt werden, die ansonsten der Wellenleiterschicht 16 nach der Figur 1 entspricht. Die Dicke der Wellenleiterschicht 23 oberhalb des zweiten, parallelen Grundelementes mit den IDT 6 ohne die Flüssigkeitsfallen kann soweit verringert werden, dass dieselbe Empfindlichkeit beider Grundelemente erreicht wird.

Eine weitere in der Figur 8 gezeigte Methode der Erzeugung von Flüssigkeitsfallen 17, bzw. Gräben 22 für Wellen des Love-Moden-Typs besteht in der Aufbringung und anschließenden Strukturierung einer weiteren Flüssigkeitsfallenschicht 25 oberhalb der akustischen Wellenleiterschicht 23, eventuell auch unter Verwendung einer zusätzlichen Zwischenschicht 21 als Haftvermittler und/oder als Ätzstopschicht ähnlich des Beispiels nach der Figur 6. Auf diese Weise wird die Reproduzierbarkeit der Grabentiefe verbessert.

Bei allen Ausführungen kann, die hier nicht dargestellte, Bildung von Flüssigkeitsfallen 17 durch Anordnung von Gruben mit kreisförmigem oder polygonalem Querschnitt oder einer schwammartigen Oberflächenstruktur, wie oben erwähnt, erfolgen. In allen beschriebenen Fällen kann oberhalb der Flüssigkeitsfallen eine dünne metallische Abschirmschicht 26, die wenige nm bis 100 nm dick sein kann, zur Abschirmung ungewollter akustoelektrischer Wechselwirkungen zwischen der Messflüssigkeit und der Sensoranordnung 1 vorgeseh werden. Bei dem Ausführungsbeispiel nach der Figur 8 kann die Zwischenschicht 21 außerdem besonders vorteilhaft gleichzeitig als Haftvermittler und Ätzstopschicht sowie als Abschirmschicht zwischen der Wellenleiterschicht 23 und den Flüssigkeitsfallen 22 genutzt werden.

Die grundsätzliche Funktionsweise der zuvor beschriebenen Sensoranordnung 1 wird im folgenden erläutert. Durch Anlegen einer Wechselspannung an die Elektroden, bzw. Wandlerfinger 7 eines der zuvor beschriebenen Interdigitaltransducers 6 werden aufgrund des inversen piezoelektrischen Effekts wechselnde mechanische Spannungen im Substrat 5 erzeugt, die eine senkrecht zu den IDT 6 durch das Substrat 5 laufende akustische Scherwelle zur Folge haben.

Ist bei Verwendung von akustischen Wellenleiterschichten, z.B. bei einer Sensoranordnung für akustische Love-Mode-Wellen, die Scherwellengeschwindigkeit in der Wellenleiterschicht 16, 23 niedriger als im Substrat 5, kommt es zu einer Konzentration der akustischen Energie unterhalb und in dieser Schicht (sog. Wellenleitereffekt). Der so entstehende Oberflächenwellentyp wird als Love-Welle bezeichnet. Diese akustischen Wellenleitermoden weisen eine vergrößerte Empfindlichkeit auf als die allgemeinen Schermoden, jedoch wird durch die Wellenleiterschicht 16, 23 auch die Ausbreitungsdämpfung der Welle beeinflusst. Bei einer Veränderung der Ausbreitungsbedingungen der akustischen Welle werden die Ausbreitungsgeschwindigkeit und die Dämpfung beeinflußt, so dass eine Messung dieser Wellenparameter Aussagen über die einwirkenden Größen liefern.

Befindet sich auf einem Sensorgrundelement mit den IDT 6 ein flüssiges Messmedium, so kommt es zu einer viskosen Kopplung, d.h. eine dünne Flüssigkeitsschicht an der Oberfläche des Sensorgrundelements wird zum Mitmachen der Scherschwingungen gezwungen. Die effektive Höhe der mitschwingenden Flüssigkeitsschicht (Abklinglänge) hängt direkt von der Viskosität und der Frequenz ab. Die viskose Kopplung bewirkt eine Abnahme der Ausbreitungsgeschwindigkeit der akustischen Welle sowie eine Zunahme der Wellendämpfung proportional zur Wurzel aus dem Dichte-Viskositätsprodukt.

Befinden sich nun auf der Oberfläche eines der Sensorgrundelemente als Flüssigkeitsfallen 17 wirkende Inhomogenitäten, so wird die Ausbreitungsgeschwindigkeit durch einen zweiten, von der Flüssigkeitsdichte und dem in den Flüssigkeitsfallen 17 eingeschlossenen Flüssigkeitsvolumen abhängigen Einfluss reduziert. Die Änderung der Ausbreitungsgeschwindigkeit kann beispielsweise gemessen werden, wenn ein Sensorgrundelement, z.B. mit der Verzögerungsleitung nach der Figur 3 oder den Reflektoren 14 nach den Figuren 4 und 5 als frequenzbestimmendes Glied in einer Oszillatorschaltung eingesetzt wird. Die Veränderung der Resonanzfrequenz eines solchen Oszillators ist ein Maß für die Geschwindigkeitsänderung der Welle.

Steht nunmehr auch noch ein Sensorgrundelement mit den IDT 6 zur Verfügung, das nicht der Flüssigkeit als Messgröße ausgesetzt ist, zur Verfügung, können durch Mischen zweier Oszillatorfrequenzen Störgrößen wie z.B. Temperatureinflüsse kompensiert werden, außerdem steht als Ausgangsgröße direkt das niederfrequente Signal Δf zur Verfügung.

In Figur 10 ist ein prinzipielles Ausführungsbeispiel eines Schaltungsaufbaus zur Ermittlung der Dichte und der Viskosität einer Messflüssigkeit mit zwei Oszillatorschaltungen 30 und 31 dargestellt. Die Oszillatorfrequenz f₁ eines Grundelementes mit den IDT 6 und Flüssigkeitsfallen 17 in der ersten Oszillatorschaltung 30 wird mit der Oszillatorfrequenz f₂ der Oszillatorschaltung 31 ohne Flüssigkeitsfallen in einem Mischer 32 gemischt, wobei die Mischfrequenz Δf am Ausgang eines nachgeschalteten Tiefpasses 33 in guter Näherung ein Maß für die Flüssigkeitsdichte ist, da der auf beide Oszillatorschaltungen 30 und 31 wirkende viskose Einfluss sowie eventuelle weitere Störgrößen kompensiert werden. Vorausgesetzt ist dabei, dass die Empfindlichkeit gegenüber viskoser Kopplung bei beiden Sensorgrundelemente identisch ist.

Die Viskosität der Messflüssigkeit kann mit der beschriebenen Schaltungsanordnung unter Benutzung der ermittelten Dichte aus der Verschiebung der Frequenz des Sensorgrundelements ohne Flüssigkeitsfallen in der Oszillatorschaltung 31 gegenüber der bekannten Frequenz bei einem Betrieb der Messanordnung ohne eine Messflüssigkeit ermittelt werden. Auf analoge Weise kann als Messgröße hier auch die Dämpfungsänderung herangezogen werden.

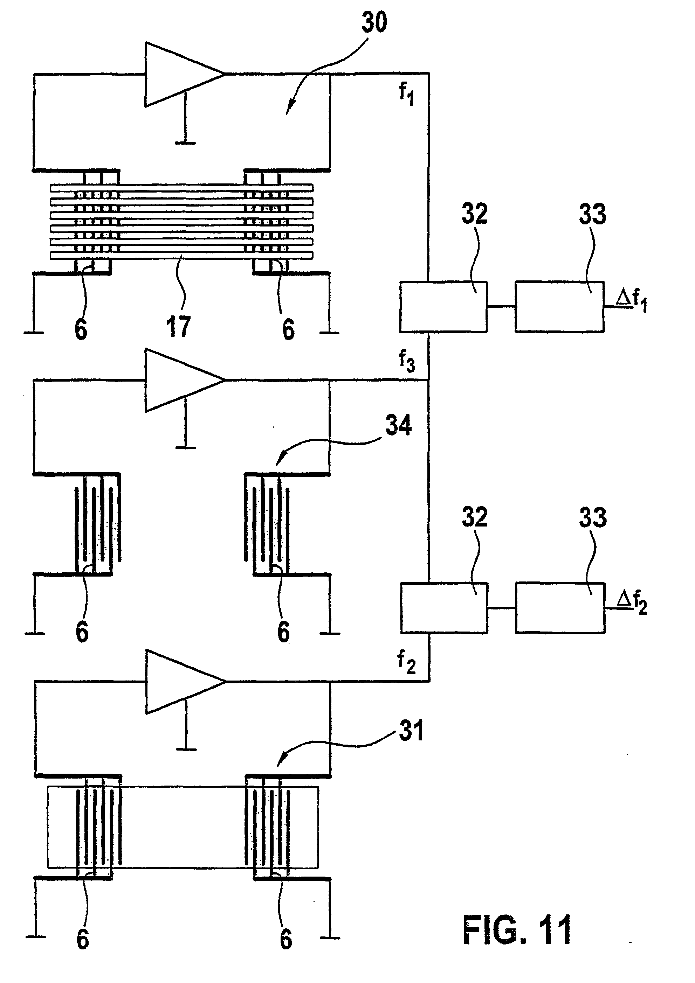

Ein weiteres, erweitertes Ausführungsbeispiel einer Schaltungsanordnung zur Erfassung der erforderlichen Messgrößen ist in Figur 11 gezeigt. In dieser Schaltungsanordnung ist zusätzlich noch eine Oszillatorschaltung 34 mit einer Messstrecke ohne die Durchleitung der Messflüssigkeit vorhanden, so dass diese Messstrecke als nicht benetztes Referenzelement dient. Diese Anordnung ist insbesondere dann vorteilhaft, wenn die beiden mit der Messflüssigkeit benetzten Sensorelemente aus technologischen Gründen keine identische Empfindlichkeit gegenüber viskosen Wechselwirkungen aufweisen. Außerdem werden bei dieser Anordnung mögliche Bauelementedriften zur Verbesserung der Langzeitstabilität homogenisiert.

Aus den Resonanzfrequenzverschiebungen Δf₁ und Δf₂ kann anhand des Aufbaus nach der Figur 11, bei bekannter Empfindlichkeit der Bauelemente gegenüber Dichte- und Viskositätsänderungen die Flüssigkeitsdichte und unter Benutzung der somit bekannten Flüssigkeitsdichte aus Δf₂ die Viskosität in der oben beschriebenen Weise ermittelt werden. Alternativ kann auch hier die Dämpfungsänderung als Messgröße genutzt werden.

Eine dritte, nicht in der Zeichnung dargestellte Ausführungsform weist zwei Sensorgrundelemente auf, die beide mit Flüssigkeitsfallen versehen sind und von denen eines mit der Messflüssigkeit und eines mit Luft in Kontakt ist. Die hier in analoger Weise zu den Ausführungsbeispielen nach den Figuren 9 bis 11 gewonnene Mischfrequenz ist dabei von der Dichte und der Wurzel aus dem Dichte-Viskositäts-Produkt abhängig. Die Dämpfungsdifferenz ist dabei auch das Maß für die Wurzel aus dem Dichte-Viskositäts-Produkt, da eine geringfügige Massezunahme durch die Messflüssigkeit in den Flüssigkeitsfallen eine vernachlässigbare Dämpfungsänderung bewirkt.

Hierbei ist somit eine Dämpfungsmessung unbedingt notwendig, allerdings ist vorteilhaft, dass nur zwei völlig identische Sensorgrundelemente notwendig sind, bei gleicher Empfindlichkeit, Drift und mechanischer Querempfindlichkeit.

## Patentansprüche

1. Sensoranordnung zur Ermittlung der Dichte und der Viskosität einer Flüssigkeit, mit
- einer Anordnung aus mindestens zwei Sensorgrundelementen, von denen mindestens eins mit der Flüssigkeit benetzbar ist und mit
- elektro-akustischen Wandlern (6) in den Sensorgrundelementen zur Erzeugung und Detektion akustischer Oberflächenwellen mit vorgegebenen Wellenmoden, aus deren Ausbreitungsverhalten entlang einer Messstrecke ein Maß für die Dichte und die Viskosität der Flüssigkeit ermittelbar ist, **dadurch gekennzeichnet, dass**
- im Bereich mindestens eines der Sensorgrundelemente parallel zur Ausbreitungsrichtung der akustischen Oberflächenwelle in der jeweiligen Messstrecke verlaufende Flüssigkeitsfallen (17) für die Flüssigkeit angeordnet sind.

2. Sensoranordnung nach Anspruch 1, **dadurch gekennzeichnet, dass**
- die ausgewerteten akustischen Oberflächenwellen horizontal polarisierte akustische Scherwellen des Love-Moden-Typs sind.

3. Sensoranordnung nach Anspruch 1 **dadurch gekennzeichnet, dass**
- die ausgewerteten akustischen Oberflächenwellen horizontal polarisierte akustische Scherwellen des SSBW- oder Leckwellen-Typs sind.

4. Sensoranordnung nach einem der Ansprüche 1 bis 3, **dadurch gekennzeichnet, dass**
- die elektro-akustischen Wandler aus auf einem Substrat (5) angeordneten Interdigitaltransducern (6) gebildet sind, deren Wandlerfinger (7) so ausgebildet sind, dass sich die erforderlichen Wellenmoden mit einer geeigneten Oszillatorfrequenz erzeugen lassen.

5. Sensoranordnung nach einem der vorhergehenden Ansprüche, **dadurch gekennzeichnet, dass**
- jeweils ein Sensorgrundelement als Verzögerungsleitung mit zwei Interdigitaltransducern (6a,6b) und einer dazwischenliegenden Ausbreitungs- oder Messstrecke (13) ausgebildet ist.

6. Sensoranordnung nach einem der Ansprüche 1 bis 4, **dadurch gekennzeichnet, dass**
- jeweils ein Sensorgrundelement als Zweitorresonator mit zwei nebeneinanderliegenden Interdigitaltransducern (6a,6b) und jeweils außen liegenden Reflektoren (14) ausgebildet ist.

7. Sensoranordnung nach einem der Ansprüche 1 bis 4, **dadurch gekennzeichnet, dass**
- jeweils ein Sensorgrundelement als Eintorresonator mit einem Interdigitaltransducer (6) und jeweils außen liegenden Resonatoren (14) ausgebildet ist.

8. Sensoranordnung nach einem der vorhergehenden Ansprüche, **dadurch gekennzeichnet, dass**
- die Flüssigkeitsfallen (17) durch Gräben oder Ätzungen (22) in einer entsprechend strukturierbaren Schicht (20) oberhalb der elektro-akustischen Wandler (6), gegebenenfalls auf einer Zwischenschicht (21), gebildet sind.

9. Sensoranordnung nach einem der Ansprüche 1 bis 7, **dadurch gekennzeichnet, dass**
- die Flüssigkeitsfallen (22) durch Gräben oder Ätzungen in einer entsprechend strukturierbaren akustischen Wellenleiterschicht (23) oberhalb der elektro-akustischen Wandler (6), gegebenenfalls mit einer äußeren metallischen Abschirmung (26), gebildet sind.

10. Sensoranordnung nach einem der Ansprüche 1 bis 7, **dadurch gekennzeichnet, dass**
- die Flüssigkeitsfallen (17) durch Gräben oder Ätzungen in einer entsprechend strukturierbaren Schicht (25) oberhalb der elektro-akustischen Wandler (6) gebildet sind und dass
- eine darunterliegenden Zwischenschicht (21) und eine weitere zwischen der Zwischenschicht (21) und dem elektro-akustischen Wandler (6) liegende akustische Wellenleiterschicht (23) vorhanden ist.

11. Sensoranordnung nach einem der vorhergehenden Ansprüche, **dadurch gekennzeichnet, dass**
- die Flüssigkeitsfallen (17) durch parallel zur Ausbreitungsrichtung der akustischen Welle verlaufende Gräben (22) gebildet sind.

12. Sensoranordnung nach einem der Ansprüche 1 bis 10, **dadurch gekennzeichnet, dass**
- die Flüssigkeitsfallen (17) aus einer Anordnung von Gruben mit kreisförmigen oder polygonalem Querschnitt oder einer schwammartigen Oberflächenstruktur gebildet sind.

13. Verfahren zur Ermittlung der Dichte und Viskosität einer Flüssigkeit mit einer Sensoranordnung nach einem der vorhergehenden Ansprüche, **dadurch gekennzeichnet, dass**
- mit einer ersten Oszillatorschaltung (30), die ein Sensorelement mit Flüssigkeitsfallen (17,22) aufweist, eine erste Oszillatorfrequenz f₁ erzeugt wird,
- mit einer zweiten Oszillatorschaltung (31), die ein Sensorelement ohne Flüssigkeitsfallen aufweist, eine zweite Oszillatorfrequenz f₂ erzeugt wird, dass
- aus der Mischfrequenz Δf der beiden Oszillatorfrequenzen f₁ und f₂ die Dichte der Flüssigkeit ermittelt wird und dass
- aus der Frequenzverschiebung der Oszillatorfrequenz f₂ der Oszillatorschaltung (31) bei einer Messung ohne Flüssigkeit gegenüber der Messung mit Flüssigkeit die Viskosität der Flüssigkeit ermittelt wird.

14. Verfahren zur Ermittlung der Dichte und Viskosität einer Flüssigkeit mit einer Sensoranordnung nach einem der Ansprüche 1 bis 12, **dadurch gekennzeichnet, dass**
- mit einer ersten Oszillatorschaltung (30), die ein Sensorelement mit Flüssigkeitsfallen (17,22) aufweist, eine erste Oszillatorfrequenz f₁ erzeugt wird, dass
- mit einer zweiten Oszillatorschaltung (31), die ein Sensorelement ohne Flüssigkeitsfallen aufweist, eine zweite Oszillatorfrequenz f₂ erzeugt wird, dass
- mit einer dritten Oszillatorschaltung (34), die ein Sensorelement ohne Flüssigkeitsfallen und ohne die zu messende Flüssigkeit aufweist, eine dritte Oszillatorfrequenz f₃ erzeugt wird, dass
- aus der Mischfrequenz Δf₁ der beiden Oszillatorfrequenzen f₁ und f₃ und der Mischfrequenz Δf₂ der beiden Oszillatorfrequenzen f₂ und f₃ die Dichte der Flüssigkeit ermittelt wird und dass
- aus der Mischfrequenz Δf₂ die Viskosität der Flüssigkeit ermittelt wird.

15. Verfahren zur Ermittlung der Dichte und Viskosität einer Flüssigkeit mit einer Sensoranordnung nach einem der Ansprüche 1 bis 12, **dadurch gekennzeichnet, dass**
- mit einer ersten Oszillatorschaltung, die ein Sensorelement mit Flüssigkeitsfallen aufweist, eine erste Oszillatorfrequenz f₁ erzeugt wird, dass
- mit einer zweiten Oszillatorschaltung, die ein Sensorelement mit Flüssigkeitsfallen und ohne die zu messende Flüssigkeit aufweist, eine zweite Oszillatorfrequenz f₂ erzeugt wird, dass
- aus der Mischfrequenz Δf der beiden Oszillatorfrequenzen f₁ und f₂ ein Messsignal in Abhängigkeit von der Dichte und der Wurzel aus dem Dichte-Viskositäts-Produkt der Flüssigkeit ermittelt wird und dass
- aus der Dämpfungsdifferenz der beiden Oszillatorfrequenzen f₁ und f₂ ein Maß für die Wurzel aus dem Dichte-Viskositäts-Produkt ermittelt wird.

## Claims

1. Sensor arrangement for determining the density and the viscosity of a liquid, having
- an arrangement and at least two basic sensor elements of which at least one can be wetted by the liquid, and having
- electroacoustic transistors (6) in the basic sensor elements for the purpose of generating and detecting acoustic surface waves with prescribed wave modes, from whose propagation behaviour along a measuring section it is possible to determine a measure of the density and the viscosity of the liquid, **characterized in that**
- in the region of at least one of the basic sensor elements, liquid traps (17) for the liquid are arranged running parallel to the direction of propagation of the acoustic surface wave in the respective measuring section.

2. Sensor arrangement according to Claim 1, **characterized in that**
- the evaluated acoustic surface waves are horizontally polarized acoustic gravity waves of the Love mode type.

3. Sensor arrangement according to Claim 1, **characterized in that**
- the evaluated acoustic surface waves are horizontally polarized acoustic gravity waves of the SSBW or leakage wave type.

4. Sensor arrangement according to one of Claims 1 to 3, **characterized in that**
- the electroacoustic transducers are formed from interdigital transducers (6) which are arranged on a substrate (5) and whose transducer fingers (7) are designed such that the required wave modes can be generated with a suitable oscillator frequency.

5. Sensor arrangement according to one of the preceding claims, **characterized in that**
- in each case a basic sensor element is designed as a delay line with two interdigital transducers (6a, 6b) and a propagation or measuring section (13) lying therebetween.

6. Sensor arrangement according to one of Claims 1 to 4, **characterized in that**
- in each case a basic sensor element is designed as a double-gate resonator with two interdigital transducers (6a, 6b) lying next to one another and reflectors (14) lying outside in each case.

7. Sensor arrangement according to one of Claims 1 to 4, **characterized in that**
- in each case a basic sensor element is designed as a single-gate resonator with an interdigital transducer (6) and reflectors (14) lying outside in each case.

8. Sensor arrangement according to one of the preceding claims, **characterized in that**
- the liquid traps (17) are formed by trenches or etched portions (22) in an appropriately structurable layer (20) above the electroacoustic transducer (6), possibly on an intermediate layer (21).

9. Sensor arrangement according to one of Claims 1 to 7, **characterized in that**
- the liquid traps (22) are formed by trenches or etched portions in an appropriately structurable acoustic waveguide layer (23) above the electroacoustic transducer (6), possibly with an outer metallic shield (26).

10. Sensor arrangement according to one of Claims 1 to 7, **characterized in that**
- the liquid traps (17) are formed by trenches or etched portions in an appropriately structurable layer (25) above the electroacoustic transducer (6), and **in that**
- an intermediate layer (21) lying therebelow and a further acoustic waveguide layer (23) lying between the intermediate layer (21) and the electroacoustic transducer (6) are present.

11. Sensor arrangement according to one of the preceding claims, **characterized in that**
- the liquid traps (17) are formed by trenches (22) running parallel to the direction of propagation of the acoustic wave.

12. Sensor arrangement according to one of Claims 1 to 10, **characterized in that**
- the liquid traps (17) are formed from an arrangement of grooves of circular or polygonal cross section, or from a sponge-like surface structure.

13. Method for determining the density and viscosity of a liquid with the aid of a sensor arrangement according to one of the preceding claims, **characterized in that**
- a first oscillator frequency f₁ is generated with the aid of a first oscillator circuit (30), which has a sensor element with liquid traps (17, 22),
- a second oscillator frequency f₂ is generated with the aid of a second oscillator circuit (31), which has a sensor element without liquid traps, **in that**
- the density of the liquid is determined from the mixed frequency Δf of the two oscillator frequencies f₁ and f₂, and **in that**
- the viscosity of the liquid is determined from the frequency shift of the oscillator frequency f₂ of the oscillator circuit (31) in the case of a measurement without liquid by comparison with the measurement with liquid.

14. Method for determining the density and viscosity of a liquid with the aid of a sensor arrangement according to one of Claims 1 to 12, **characterized in that**
- a first oscillator frequency f₁ is generated with the aid of a first oscillator circuit (30), which has a sensor element with liquid traps (17, 22), **in that**
- a second oscillator frequency f₂ is generated with the aid of a second oscillator circuit (31), which has a sensor element without liquid traps, **in that**
- a third oscillator frequency f₃ is generated with the aid of a third oscillator circuit (34), which has a sensor element without liquid traps and without the liquid to be measured, **in that**
- the density of the liquid is determined from the mixed frequency Δf₁ of the two oscillator frequencies f₁ and f₃ and from the mixed frequency Δ₂, and **in that**
- the viscosity of the liquid is determined from the mixed frequency Δf₂.

15. Method for determining the density and the viscosity of a liquid with the aid of a sensor arrangement according to one of Claims 1 to 12, **characterized in that**
- a first oscillator frequency f₁ is generated with the aid of a first oscillator circuit, which has a sensor element with liquid traps, **in that**
- a second oscillator frequency f₂ is generated with the aid of a second oscillator circuit, which has a sensor element with liquid traps and without the liquid to be measured, **in that**
- a measuring signal is determined from the mixed frequency Δf of the two oscillator frequencies f₁ and f₂ as a function of the density and the root of the density/viscosity product of the liquid, and **in that**
- a measure of the root of the density/viscosity product is determined from the damping difference between the two oscillator frequencies f₁ and f₂.

## Revendications

1. Dispositif de capteur pour déterminer la densité et la viscosité d'un liquide, comprenant :
- un montage formé de deux éléments de base de capteur dont au moins l'un peut être mouillé par le liquide, et
- des convertisseurs électro-acoustiques (6) dans les éléments de base des capteurs pour générer et détecter des ondes acoustiques de surface avec des modes d'ondes prédéterminés, permettant à partir de leur comportement en extension le long d'un chemin de mesure, de déterminer une mesure de la densité et de la viscosité du liquide,
**caractérisé en ce que**
- au niveau d'au moins l'un des éléments de base de capteur en parallèle à la direction d'étalement de l'onde acoustique de surface dans le chemin de mesure respectif on a des puits à liquide (17) pour recevoir le liquide.

2. Dispositif de capteur selon la revendication 1,
**caractérisé en ce que**
les ondes acoustiques de surface, exploitées, sont des ondes de cisaillement acoustiques, à polarisation horizontale, du type mode Love.

3. Dispositif de capteur selon la revendication 1,
**caractérisé en ce que**
les ondes acoustiques de surface, exploitées, sont des ondes de cisaillement acoustiques à polarisation horizontale, du type SSBW ou du type ondes de fuite.

4. Dispositif de capteur selon l'une quelconque des revendications 1 à 3,
**caractérisé en ce que**
les convertisseurs électro-acoustiques sont formés d'un substrat (5) à transducteurs interdigitaux (6) dont les doigts de convertisseur (7) sont conçus pour pouvoir gérer les modes d'ondes nécessaires avec une fréquence d'oscillateur appropriée.

5. Dispositif de capteur selon l'une quelconque des revendications précédentes,
**caractérisé en ce que**
chaque fois un élément de base de capteur est réalisé comme ligne de retard avec chaque fois deux transducteurs interdigitaux (6a, 6b) et entre ceux-ci un chemin d'extension ou de mesure (13).

6. Dispositif de capteur selon l'une quelconque des revendications 1 à 4,
**caractérisé en ce que**
chaque fois un élément de base de capteur est réalisé comme résonateur double avec deux transducteurs interdigités (6a, 6b) juxtaposés et chaque fois des réflecteurs (14) réalisés à l'extérieur.

7. Dispositif de capteur selon l'une quelconque des revendications 1 à 4,
**caractérisé en ce que**
chaque fois un élément de capteur de base est réalisé comme résonateur à une porte avec un transducteur interdigital (6) et chaque fois un résonateur (14) situé à l'extérieur.

8. Dispositif de capteur selon l'une quelconque des revendications précédentes,
**caractérisé en ce que**
les puits de liquide (17) sont formés par des sillons ou des gravures (22) dans une couche à structure correspondante (20) au-dessus du convertisseur électro-acoustique (6), le cas échéant sur une couche intermédiaire (12).

9. Dispositif de capteur selon l'une quelconque des revendications 1 à 7,
**caractérisé en ce que**
les puits à liquide (22) sont réalisés par gravure ou corrosion dans une couche d'ondes acoustiques de surface (23), structurée de façon correspondante, au-dessus du convertisseur électro-acoustique (6), le cas échéant muni d'un écran extérieur métallique (26).

10. Dispositif de capteur selon l'une quelconque des revendications 1 à 7,
**caractérisé en ce que**
les puits de liquide (17) sont formés par des sillons ou des gravures dans une couche (25) structurée de manière correspondante au-dessus des convertisseurs électro-acoustiques (6), avec
une couche intermédiaire (21) située en dessous et une autre couche guide d'ondes acoustiques (23) entre la couche intermédiaire (21) et le convertisseur électro-acoustique (6).

11. Dispositif de capteur selon l'une quelconque des revendications précédentes,
**caractérisé en ce que**
les puits à liquide (17) sont formés par des sillons (22) parallèles à la direction d'expansion de l'onde acoustique.

12. Dispositif de capteur selon l'une quelconque des revendications 1 à 10,
**caractérisé en ce que**
les puits à liquide (17) sont formés par une disposition de sillons à section circulaire ou polygonale ou par une structure de surface en forme de peigne.

13. Procédé pour déterminer la densité et la viscosité d'un liquide avec un dispositif de capteur selon l'une quelconque des revendications précédentes,
**caractérisé en ce qu'**
- on génère une première fréquence d'oscillateur (f₁) avec un premier circuit à oscillateur (30) ayant un élément de capteur avec un puits de liquide (17, 22),
- on génère une seconde fréquence d'oscillation (f₂), avec un second oscillateur (31), comportant un élément de capteur sans puits à liquide,
- à partir de la fréquence de mélange (Δf) des deux fréquences d'oscillateur (f₁, f₂) on détermine la densité du liquide, et
- à partir du décalage de fréquence de la fréquence (f₂) de l'oscillateur (31), pour la mesure sans liquide par rapport à la mesure avec du liquide, on détermine la viscosité du liquide.

14. Procédé pour déterminer la densité et la viscosité d'un liquide à l'aide d'un dispositif de capteur selon l'une quelconque des revendications 1 à 12,
**caractérisé en ce qu'**
- on génère une première fréquence d'oscillateur (f₁), avec un premier oscillateur (30) comportant un élément de capteur avec des puits à liquide (17, 22)
- avec un second oscillateur (31) comportant un élément de capteur sans puits à liquide on génère une seconde fréquence d'oscillateur (f₂),
- on génère une troisième fréquence d'oscillateur, avec un troisième oscillateur (34) comportant un élément de capteur sans puits à liquide et sans liquide à mesurer, et
- à partir de la fréquence mélangée (Δf₁) des deux fréquences d'oscillateur (f₁) et (f₃) et de la fréquence mélangée (Δf₂) des deux fréquences d'oscillateur (f₂, f₃) on détermine la densité du liquide, et
à partir de la fréquence mélangée (Δf₂) on détermine la viscosité du liquide.

15. Procédé pour déterminer la densité et la viscosité d'un liquide avec un dispositif de capteur selon l'une quelconque des revendications 1 à 12,
**caractérisé en ce qu'**
- on génère une seconde fréquence d'oscillateur (f₂), avec un premier oscillateur comportant un élément de capteur avec des puits à liquide on génère une première fréquence d'oscillateur (f₁), avec un second oscillateur comportant un élément de capteur avec des puits à liquide et sans liquide à mesurer,
- à partir de la fréquence mélangée (Δf) des deux fréquences (f₁, f₂) d'oscillateur, on génère un signal de mesure en fonction de la densité et de la racine du produit densité/viscosité du liquide et
- à partir de la différence d'amortissement des deux fréquences d'oscillateur (f₁, f₂) on détermine une mesure de la racine du produit densité/viscosité.
